# EUROPÄISCHE PATENTANMELDUNG

(11) **EP 1 944 023 A1**
(43) Veröffentlichungstag der Anmeldung: **16.07.2008**
(21) Anmeldenummer: 08000516.8
(22) Anmeldetag: 11.01.2008
(51) Int. Cl.: A61K 31/164, A61K 36/484, A61K 36/49, A61P 31/22

(54) **Produkt Süssholzwurzel + Eichenrinde in Kombination mit Dexpanthenol**

(30) Priorität: 15.01.2007 DE 102007002170
(71) Anmelder: Geske, Gundula, 73277 Owen (DE)
(72) Erfinder: Geske, Gundula, 73277 Owen (DE)
(74) Vertreter: Dey, Michael

(57) **Zusammenfassung**

Die Erfindung betrifft eine pharmazeutische Zusammensetzung, umfassend einen Extrakt aus Süßholzwurzel und Eichenrinde in Kombination mit Dexpanthenol, sowie deren Verwendung zur Behandlung von Erkrankungen, welche durch Mikroorganismen hervorgerufen werden, und als Kosmetikum.

## Beschreibung

Die Erfindung betrifft eine pharmazeutische Zusammensetzung, umfassend einen Extrakt aus Süßholzwurzel und Eichenrinde in Kombination mit Dexpanthenol, sowie deren Verwendung zur Behandlung von Erkrankungen, welche durch Mikroorganismen hervorgerufen werden, und als Kosmetikum.

Entzündungen im Lippen- und Rachenraum sind ein sehr verbreitetes Problem. Diese Entzündungen gehen häufig auf virale Infektionen zurück. Verursacher solcher Infektionen sind oft die sogenannten Herpes-Viren. Hierbei gibt es viele verschiedene Arten dieser Viren, wobei nur einige davon beim Menschen eine Krankheit auslösen.

Obwohl diese verschiedenen Herpes-Viren sich unterschiedlich verhalten, ist ihnen allen eine unangenehme Eigenschaft eigen: wenn sie einmal in den Körper eingedrungen sind und eine Erstinfektion verursacht haben, verlassen sie den Körper nicht mehr. Vielmehr ziehen sie sich an den Nervenfasern entlang in die Nervenknoten als ruhende "latente" Viren zurück. Unter bestimmten Umständen können dann diese Viren erneut aktiv werden und ein Krankheitsbild hervorrufen.

Ein besonders weit verbreitetes, unangenehmes Problem stellt eine Herpes-Infektion dar, die sich in Form von Bläschen an den Lippen, dem sogenannten Herpes labialis, äussert. Dies ist die häufigste Form einer Herpes-Erkrankung. Die Infektion beruht auf dem Herpes-Simplex-Virentyp 1. Etwa 30 bis etwa 70% der Bevölkerung werden bereits im Kindesalter mit diesem Virus infiziert. Allerdings treten die Symptome nicht zwangsläufig bei den Infizierten auf. Die Viren setzen sich nach der Primärinfektion in den Nervenknoten fest und verursachen bei vielen Betroffenen von Zeit zu Zeit die sehr unangenehmen sogenannten Herpes-Bläschen, die vorwiegend an den Lippen auftreten. Diese sogenannten Rezidive erscheinen vornehmlich unter bestimmten Umständen, beispielsweise unter Stress, extremer Sonnenbestrahlung, Hautreizung oder anderen Belastungen. Zunächst macht sich hierbei ein Juckreiz und ein Spannungsgefühl bemerkbar. Sodann treten gruppierte Bläschen auf gerötetem Grund auf, die später zu Krusten eintrocknen und abheilen.

Herpes-Viren können jedoch auch ganz andere Krankheitsbilder verursachen. Beispielsweise wird die Kinderkrankheit Windpocken gleichfalls von Herpes-Viren verursacht. Die Viren verbleiben als latente Viren im Körper und können viele Jahre später wieder aktiv werden und eine sogenannte Gürtelrose, den Herpes zoster, hervorrufen. Weiterhin können sich Herpes-Infektionen auch im Auge manifestieren oder sich als Herpes der Geschlechtsorgane oder virale Hirnhautentzündung äussern.

Es gibt eine Fülle von Hausrezepten, die bei Herpes-Erkrankungen, insbesondere Herpes labialis, empfohlen werden. Im Allgemeinen sind diese Rezepte jedoch wenig wirksam. Bis zu einem gewissen Umfang hat sich der Wirkstoff Aciclovir als wirksam bei der Behandlung von Herpes-Bläschen an den Lippen erwiesen. Eine Therapie mit Aciclovir ist jedoch in jedem Fall rein symptomatisch, so dass es immer wieder zum Ausbruch von Rezediven, also einer Reaktivierung der latenten Viren, kommt.

In Anbetracht eines kontinuierlich steigenden Bedarfs, für die Behandlung von Erkrankungen, welche durch Mikroorganismen, und insbesondere Viren, hervorgerufen werden, Alternativen zu synthetischen Produkten mit gleichem oder ähnlichem Wirkspektrum bereitzustellen, wurden in den vergangenen Jahren auch verschiedene Erzeugnisse beschrieben, welche auf natürlich vorkommenden Produkten basieren.

Die japanische Patentanmeldung JP 07179354 offenbart ein antivirales Mittel, welches einen wässrigen Extrakt einer Mischung aus zehn verschiedenen pflanzlichen Ausgangsstoffen, darunter Süßholzwurzel (Glycyrrhiza glabra) und Eichenrinde (Quercus spec.), enthält.

EP 0 568 001 A2 beschreibt ein Mittel, umfassend wenigstens einen wässrigen oder methanolischen Extrakt einer Rohdroge wie beispielsweise Süßholzwurzel und Eichenrinde, zur Behandlung von viralen Erkrankungen, welche unter anderem durch Cytomegalieviren hervorgerufen werden.

Die europäische Patentanmeldung EP 1 238 672 A1 beschreibt ein pharmazeutisches Kombinationspräparat, welches einen wässrigethanolischen Extrakt von Süßholzwurzel und Eichenrinde enthält und zur Behandlung von viralen Erkrankungen wie beispielsweise Herpes labialis verwendet werden kann.

Vorstehend genannte Zusammensetzungen sind jedoch mit einer Reihe von Nachteilen behaftet. JP 07179354 und EP 0 568 001 A2 offenbaren jeweils eine Extraktion von Rohdrogen in siedendem Lösungsmittel. Die Herstellung des Extrakts aus Süßholzwurzel und Eichenrinde gemäß EP 1 238 672 A1 erfolgt bei Temparaturen von 10 bis 80°C, wobei Raumtemperatur und ein Bereich von 45 bis 55°C als gleichermaßen bevorzugt angesehen werden.

In Anbetracht der Tatsache, dass viele Naturstoffe die oben genannten hohen Temperaturen nicht tolerieren und sich dabei entweder zersetzen oder verflüchtigen, werden einem auf diese Weise hergestellten Extrakt zwangsläufig zahlreiche Wirkstoffe vollständig entzogen, womit sich das Wirkspektrum eines solchen Extrakts von einem bei niedrigeren Temperaturen bzw. schonenderen Extraktionsbedingungen hergestellten Extrakts in der Regel deutlich unterscheidet.

Ferner weist das in EP 1 238 672 A1 offenbarte Kombinationspräparat Defizite hinsichtlich der Verträglichkeit auf. So wird gemäß vorstehender Patentanmeldung ein wässrig-ethanolischer Extrakt bereitgestellt, welcher ohne Zusatz weiterer Stoffe als pharmazeutisches Präparat Anwendung findet. Andererseits bewirkt der hohe Ethanolanteil des Kombinationspräparats ein rasches Austrocknen der Schleimhäute, was für Anwendungen, wie sie in EP 1 238 672 A1 beschrieben sind, allgemein unerwünscht ist.

Somit war es eine Aufgabe der vorliegenden Erfindung, ein Produkt bereitzustellen, welches die Probleme, die mit den im Stand der Technik beschriebenen Zusammensetzungen verbunden sind, zumindest teilweise löst. Diese Aufgabe wird erfindungsgemäß gelöst durch eine pharmazeutische Zusammensetzung, umfassend einen Extrakt aus Süßholzwurzel und Eichenrinde in Kombination mit Dexpanthenol, welche in geeigneter Form appliziert wird.

In einem ersten Aspekt betrifft die vorliegende Erfindung damit eine pharmazeutische Zusammensetzung, umfassend einen Extrakt aus Süßholzwurzel und Eichenrinde in Kombination mit Dexpanthenol.

Überraschenderweise wurde im Rahmen der vorliegenden Erfindung festgestellt, dass eine Zusammensetzung, welche neben einem Extrakt aus Süßholzwurzel und Eichenrinde zusätzlich Dexpanthenol enthält, zur Behandlung von Erkrankungen, welche durch Mikroorganismen hervorgerufen werden, besonders gut geeignet ist. Dabei wurde festgestellt, dass synergistische Effekte obiger Bestandteile zu einer deutlich verbesserten Wirkung bzw. Verträglichkeit der erfindungsgemäßen Zusammensetzung gegenüber den im Stand der Technik offenbarten Präparaten führen.

Der Begriff "Extrakt aus Süßholzwurzel und Eichenrinde", wie er im Rahmen der vorliegenden Anmeldung verwendet wird, bezeichnet sowohl einen Extrakt, welcher durch Extraktion einer Mischung aus Süßholzwurzel und Eichenrinde erhalten wird, als auch einen Extrakt, welcher ein Gemisch eines Extraktes aus Süßholzwurzel und eines Extraktes aus Eichenrinde darstellt. Obwohl beide Varianten der Extraktion möglich und durch vorliegende Erfindung umfasst sind, ist eine gemeinsame Extraktion der beiden Bestandteile Süßholzwurzel und Eichenrinde zu bevorzugen.

Ein erster Bestandteil der erfindungsgemäßen pharmazeutischen Zusammensetzung ist Süßholzwurzel. Als Süßholzwurzel kommen die getrockneten Wurzeln der Süßholzpflanze (Glycyrrhiza glabra) zum Einsatz. Diese Pflanze ist der Familie der Schmetterlingsblütler zuzuordnen und kommt vor allem wildwachsend als Staudenpflanze von Südeuropa bis Westsibirien, Südamerika, Vorder- und Mittelasien sowie Australien vor. Weiterhin wird Süßholz auch auf warmen sandigen Böden vor allem in Osteuropa angebaut. Die Planze ist holzig und kann bis zu 2 m hoch werden. Zur Ernte der Wurzel werden nach 3 bis 4 Jahren die bis zu 2 cm dicken Nebenwurzeln abgeschnitten und getrocknet. Die Ernte kann alle drei Jahre vom Spätherbst bis zum Frühjahr erfolgen. Die Droge hat einen kaum wahrnehmbaren Geruch und schmeckt ausserordentlich süss. Die Wurzel und auch der Saft der Süßholzpflanze finden schon lange in der Medizin Verwendung. Beispielsweise wird die getrocknete Wurzel als Teemischung eingesetzt. Ein konzentrierter Extrakt der Süßholzwurzel soll bei der Behandlung von Magengeschwüren hilfreich sein. Besondere Verbreitung hat der eingedickte Süßholzsaft als wichtiger Bestandteil von Lakritzwaren erlangt.

Eichenrinde, welche den zweiten Bestandteil der erfindungsgemäßen Zusammensetzung darstellt, kommt die getrocknete Rinde junger Stämme und Zweige der Stiel- oder Sommereiche (Quercus robur) und der Stein- oder Wintereiche (Quercus petraea) zum Einsatz. Die Eichenrinde ist vor allem aus ost- und südosteuropäischen Ländern lieferbar, wo sie angebaut wird. Die Ernte der Rinde wird in der Regel von März bis April, also vor der Entwicklung der Blätter, bei einem Alter von etwa 3 bis etwa 10 Jahren der Bäume vorgenommen. Vor allem aufgrund des hohen Gerbstoffgehaltes wird die Eichenrinde als medizinisch wirksame Droge seit langem eingesetzt. Die hierdurch bedingte adstringierende Wirkung macht sie als Badezusatz zur Linderung von entzündlichen Hauterkrankungen und als Mittel zur Behandlung von Durchfallerkrankungen geeignet.

Als dritter Bestandteil der erfindungsgemäßen pharmazeutischen Zusammensetzung findet Dexpanthenol Anwendung, welches im Körper von Säugern zu Pantothensäure (Vitamin B5) metabolisiert wird. Als Baustein des Coenzyms A ist Pantothensäure seinerseits an zahlreichen wichtigen Stoffwechselvorgängen beteiligt und für die Erhaltung und Regeneration von Gewebe maßgeblich.

In Übereinstimmung mit der Erfindung umfassen die in vorliegender Anmeldung offenbarten Zusammensetzungen die oben beschriebene Kombination von Extrakt und Dexpanthenol in einer Menge von etwa 1 bis etwa 100%, stärker bevorzugt von etwa 50 bis etwa 100%, und am stärksten bevorzugt von etwa 90 bis etwa 100%, bezogen auf das Gesamtgewicht der pharmazeutischen Zusammensetzung. Erfindungsgemäß kann das Gewichtsverhältnis von Extrakt zu Dexpanthenol zwischen etwa 99:1 und etwa 90:10 liegen, wobei ein Bereich zwischen etwa 98:2 und etwa 95:5 bevorzugt ist.

Der Extrakt aus Süßholzwurzel und Eichenrinde wird vorteilhafterweise mit Hilfe von Auszugs- bzw. Extraktionsmitteln gewonnen, die in der Herstellung pflanzlicher Extrakte üblich sind. Bevorzugt ist hierbei der Einsatz von Extraktionspaaren, wie beispielsweise einem Lösungsmittelgemisch aus Wasser und einem organischen Lösungsmittel. In einer bevorzugten Ausführungsform handelt es sich bei dem organischen Lösungsmittel um einen aliphatischen Alkohol mit 1 bis 6 Kohlenstoffatomen, stärker bevorzugt um einen aliphatischen Alkohol mit 2 bis 4 Kohlenstoffatomen, und am stärksten bevorzugt um Ethanol. Es werden jedoch auch beispielsweise Ölextrakte oder Presssäfte der pflanzlichen Drogen von der Erfindung mit umfasst. Besonders bevorzugt sind Auszugsmittel, die lipophile und hydrophile Bestandteile der pflanzlichen Drogen extrahieren.

Als überaus vorteilhaftes Auszugs- bzw. Extraktionsmittel hat sich im Rahmen der vorliegenden Erfindung ein Lösungsmittelgemisch aus Wasser und Ethanol erwiesen, in welchem der Volumenanteil von Ethanol auf etwa 20 bis etwa 80% eingestellt ist. Stärker bevorzugt beträgt der Volumenanteil von Ethanol etwa 40 bis etwa 70%, am stärksten bevorzugt etwa 45 bis etwa 55%. Bei dem zur Herstellung des Lösungsmittelgemisches verwendeten Wasser kann es sich um deionisiertes Wasser oder aber auch normales Leitungswasser handeln.

Die Extraktion der pflanzlichen Drogen wird erfindungsgemäß in einem Temperaturbereich von etwa 15 bis etwa 35°C durchgeführt. Bevorzugt sind Temperaturen von etwa 20 bis etwa 30°C, am stärksten bevorzugt von etwa 25°C (Raumtemperatur). Unter diesen Bedingungen findet eine ausreichende Extraktion der pflanzlichen Inhaltsstoffe unter sehr praktikablen und schonenden Bedingungen statt.

In einer bevorzugten Ausführungsform der Erfindung wird die Extraktion ruhend durchgeführt, d.h. dass der Ansatz während der Extraktion mit einem geeigneten Extraktionsmittel nicht bewegt wird. Es ist jedoch durchaus möglich und unter Umständen vorteilhaft, dass während der Extraktion der Ansatz bewegt, beispielsweise gerührt oder geschüttelt wird.

Die Extraktion wird vorteilhafterweise über mehrere Stunden hinweg durchgeführt. Bevorzugte Zeiten hierbei sind etwa 2 bis etwa 48 Stunden. Die Dauer der Extraktionszeit kann in einer gewissen Wechselwirkung mit der gewählten Extraktionstemperatur stehen. So kann es vorteilhaft sein, eine Extraktion bei niedrigeren Temperaturen über einen längeren Zeitraum hinweg durchzuführen. Beispielsweise kann eine Extraktion bei Raumtemperatur über 24 Stunden hinweg, und eine Extraktion bei einer Temperatur von 40°C über 8 Stunden hinweg vorteilhaft sein. In der Regel werden jedoch auch bei kürzeren Extraktionszeiten ausreichende Mengen der Inhaltsstoffe der pflanzlichen Drogen extrahiert und auch längere Extraktionszeiten sind im allgemeinen nicht nachteilig.

Für den Extrakt aus Süßholzwurzel und Eichenrinde werden etwa 1 bis etwa 5 Teile Süßholzwurzel und etwa 1 bis etwa 3 Teile Eichenrinde verwendet. Diese Teile beziehen sich auf die Gewichte der pflanzlichen Drogen. Auf diese Weise kann das Gewichtsverhältnis von Süßholzwurzel zu Eichenrinde bei der Herstellung des Extrakts erfindungsgemäß von etwa 5:1 bis etwa 1:3 betragen. In einer besonders bevorzugten Ausführungsform der Erfindung liegt das Gewichtsverhältnis von Süßholzwurzel zu Eichenrinde bei etwa 2:1. Dieses Verhältnis der beiden Drogen hat sich in Bezug auf die Wirkung als besonders vorteilhaft erwiesen.

Für die Herstellung des Extraktes können die üblichen Aufbereitungsformen der Drogen eingesetzt werden. Vorteilhafterweise werden Süßholzwurzel und Eichenrinde in getrockneter und/oder geschnittener Form eingesetzt. Diese sogenannten Schnittdrogen werden bevorzugt in einer Größe von etwa Linsen oder Bohnen verwendet. Dies hat den Vorteil, dass die pflanzlichen Teile nach erfolgter Extraktion problemlos im Ansatz sedimentieren und der Extrakt einfach abgegossen oder grobfiltriert werden kann. Es ist jedoch auch möglich, dass die Drogen als Pulver, also als gemahlene Pflanzenteile, eingesetzt werden. Für die weitere Aufarbeitung nach der Extraktion ist in diesem Fall in der Regel eine Filtration notwendig.

Der nach der Extraktion gewonnene Auszug kann auf verschiedene Weise weiterbearbeitet werden. Beispielsweise kann der Auszug aufkonzentriert werden, um so eine höhere Wirkstoffkonzentration zu erreichen. Im Allgemeinen ist die Wirkstoffkonzentration des Auszugs nach Extraktion jedoch so hoch, dass eine weitere Aufkonzentration unterbleiben und der Extrakt nach Kombination mit Dexpanthenol und gegebenenfalls weiteren Additiven, etc. ohne weitere Verarbeitung in flüssiger Form von einem Verbraucher angewendet bzw. an einen Patienten verabreicht werden kann. Dies ist besonders vorteilhaft, da die Herstellung der Zusammensetzung auf diese Weise ausgesprochen einfach und kostengünstig ist. Der flüssige Auszug kann daher beispielsweise ohne weiteres als Tinktur auf die Lippen oder im Rachenraum aufgetragen werden.

Es ist weiterhin möglich, die erfindungsgemäße pharmazeutische Zusammensetzung mindestens teilweise in getrockneter Form bereitzustellen. Hierfür wird das Extraktionsmittel nach der Extraktion durch Verdampfung, beispielsweise durch Gefriergetrocknung, entfernt, so dass die extrahierten Inhaltsstoffe der Drogen in getrockneter Form vorliegen. Diese Inhaltsstoffe können dann in verschiedener Weise weiterverwendet oder weiterverarbeitet werden.

Erfindungsgemäß enthält die pharmazeutische Zusammensetzung den Extrakt aus Süßholzwurzel und Eichenrinde in Kombination mit Dexpanthenol bevorzugt zusammen mit einem oder mehreren pharmazeutisch annehmbaren Trägern, Feuchthaltemittel und/oder Additiven, wobei der Ausdruck "pharmazeutisch annehmbarer Träger" einen oder mehrere flüssige, halbfeste oder feste Verdünnungsmittel, Füllstoffe oder andere Substanzen bezeichnet, welche für eine Verabreichung an Säuger, einschließlich Menschen, geeignet sind.

Der hier verwendete Ausdruck "pharmazeutisch annehmbar" bezeichnet jegliches nicht-toxische Material, welches die Wirksamkeit der biologischen Aktivität des Wirkstoffes nicht beeinträchtigt. Derartige Materialien können pharmazeutisch annehmbare Konzentrationen an Salzen, Puffern, Konservierungsstoffen oder dergleichen umfassen, wobei es sich bei den Salzen im Falle medizinischer Anwendungen um pharmazeutisch annehmbare Salze handeln sollte. Eine Anwendung von nicht pharmazeutisch annehmbaren Salzen ist dahingehend denkbar, sofern aus derartigen Salzen pharmazeutisch annehmbare Salze hergestellt werden können.

Der Ausdruck "Träger" im Sinne der vorliegenden Erfindung bezeichnet jeglichen organischen oder anorganischen, natürlichen oder synthetischen Stoff, welcher zur Vereinfachung der Applikation mit dem Wirkstoff kombiniert werden kann. Beispiele für derartige Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

In einer bevorzugten Ausführungsform dient der pharmazeutisch annehmbare Träger als Nahrungsmittel, Genussmittel und/oder Körperpflegemittel. So kann die Kombination von Extrakt und Dexp beispielsweise in ein Kaubonbon, in ein Kaugummi oder auch in Zahnpasta eingearbeitet sein. So gelangen die wirksamen Inhaltsstoffe der erfindungsgemäßen Zusammensetzung durch Kauen oder Lutschen bzw. durch Zähneputzen an den Ort der Erkrankung und können hier ihre Wirkung entfalten. Die Einarbeitung in ein Kaugummi hat beispielsweise den Vorteil, dass ein solches Kaugummi über einen relativ langen Zeitraum im Mund- bzw. Rachenraum verbleibt und so die wirksamen Inhaltsstoffe der Zusammensetzung hier ihre Wirkung langfristig entfalten können.

Erfindungsgemäß kann die pharmazeutische Zusammensetzung der vorliegenden Anmeldung weiterhin ein oder mehrere geeignete Feuchthaltemittel umfassen. Bevorzugte Beispiele für Feuchthaltemittel sind pharmazeutisch verträgliche Polyalkohole, wie etwa Propylenglykol, insbesondere 1,2-Propylenglykol, Pentylenglykol, insbesondere 1,2-Pentandiol, Glycerin und/oder Polyethylenglykol.

Additive im Sinne der vorliegenden Erfindung umfassen beispielsweise Reagenzien zur Einstellung des pH-Werts, Puffer, Verdünnungsmittel, Verarbeitungshilfsmittel wie etwa Emulgatoren, Konservierungsmittel, Stabilisatoren, Antioxidationsmittel, Lichtschutzmittel, Farbstoffe und dergleichen. Als Reagenzien zur Einstellung des pH-Werts werden bevorzugt insbesondere basisch reagierende Verbindungen von Alkali- oder Erdalkalimetallen, wie beispielsweise Hydroxide, Hydrogencarbonate, Carbonate, etc. verwendet, was unter anderem auch metallorganische Verbindungen umfasst. Gleichfalls ist es möglich, gegebenenfalls auch Säuren, umfassend organische und anorganische Säuren, zur Einstellung des pH-Werts einzusetzen. Geeignete Puffer umfassen, sind jedoch nicht beschränkt auf, Essigsäure, Zitronensäure, Weinsäure, Borsäure oder Phosphorsäure in Kombination mit ihren korrespondierenden Basen. Konservierungsstoffe, welche im Rahmen der vorliegenden Erfindung verwendet werden können, umfassen, sind jedoch nicht beschränkt auf, Benzalkoniumchlorid, Chlorbutanol, Thiomersal oder Parabene.

Die erfindungsgemäßen pharmazeutischen Zusammensetzungen sind bevorzugt als Spray, Gel, Creme, Salbe, Lotion, Tablette oder Zäpfchen formuliert. Die Zusammensetzungen können jedoch auch in Form von Aerosolen, wässrigen oder nicht-wässrigen Lösungen, Schäumen, Emulsionen, Suspensionen, oder anderen geeigneten Formulierungen appliziert werden.

Die Verabreichung der erfindungsgemäßen Zusammensetzungen erfolgt bevorzugt topisch. Diese topische Verabreichung ist besonders für die Behandlung von Herpes-Bläschen oder von lokalisierbaren Entzündungen im Rachenraum geeignet. Die Auftragung kann hierbei mit dem Finger oder mit anderen Hilfsmitteln vorgenommen werden. Besonders bevorzugt ist eine Auftragung mit Hilfe eines Applikators, da hierdurch Verunreinigungen der pharmazeutischen Zusammensetzung vermieden werden. Beispielsweise kann die Zusammensetzung mit einem Zellstofftupfer, insbesondere einem herkömmlichen Wattestäbchen, aufgetragen werden. Es sind jedoch auch andere Applikatoren durchaus möglich. Besonders vorteilhaft ist hierbei, wenn der Applikator ein gewisses Flüssigkeitsreservoir aufweist.

Andere Verabreichungsformen, welche für eine Applikation von Zusammensetzungen im Sinne der vorliegenden Erfindung geeignet sind, sind einem Fachmann auf dem Gebiet der Pharmakologie bzw. Medizin bekannt und umfassen, sind jedoch nicht beschränkt auf, subkutan, intradermal, transdermal, oral, nasal, inhalativ, rektal oder intravenös.

Die erfindungsgemäßen Zusammensetzungen werden derart verabreicht, dass pro Verabreichungsdosis bevorzugt eine Menge im Bereich von etwa 10 mg bis etwa 10 g, und besonders bevorzugt eine Menge im Bereich zwischen etwa 100 mg bis etwa 1 g der Kombination aus Extrakt und Dexpanthenol in getrockneter Form bereitgestellt wird. In einer bevorzugten Ausführungsform handelt es sich bei der verabreichten Menge um eine pharmazeutisch wirksame Menge.

Erfindungsgemäß dienen die in vorliegender Anmeldung beschriebenen Zusammensetzungen der Behandlung von Erkrankungen, welche durch Mikroorganismen hervorgerufen werden. Der hier verwendete Ausdruck "Behandlung" bezeichnet dabei eine therapeutische Behandlung, in welcher dem Empfänger eine zur Vorbeugung, Linderung oder Beseitigung der Erkrankung oder Störung effektive Menge der erfindungsgemäßen Zusammensetzungen verabreicht wird.

Der Ausdruck "Mikroorganismen" im Sinne der vorliegenden Erfindung umfasst Bakterien, Pilze, Hefen, Protozoen, Algen und Viren. Bevorzugt handelt es sich bei den die Erkrankung hervorrufenden Mikroorganismen um Bakterien, Pilze oder Viren, am stärksten bevorzugt um Viren.

Wie anhand des Beispiels der vorliegenden Erfindung gezeigt wird, besitzt die erfindungsgemäße Zusammensetzung eine äußerst überraschende positive Wirkung bei der Behandlung insbesondere viraler Erkrankungen. Der beobachtbare synergistische Effekt der Bestandteile der erfindungsgemäßen pharmazeutischen Zusammensetzung beruht vermutlich auf einer gegenseitigen Unterstützung von virulogischen und immunologischen Effekten der verschiedenen Inhaltsstoffe von Süßholzwurzel und Eichenrinde sowie Dexpanthenol.

Bei einer viralen Infektion wechselwirken die Viren mit Oberflächenrezeptoren von Zellen, woraufhin die Viren in die Zellen eindringen können. Innerhalb der Zellen vermehren sich die Viren auf Kosten der Zellen und verlassen die Zelle anschließend, wobei die Zelle zerstört wird. Einer der Effekte der erfindungsgemäßen pharmazeutischen Zusammensetzung ist es vermutlich, dass die Oberflächenrezeptoren geschädigt werden, so dass eine Anheftung der Viren verhindert und auf diese Weise das Eindringen der Viren in die Zelle nicht stattfinden kann. Bei dieser Schädigung der Oberflächenrezeptoren handelt es sich vermutlich um eine unspezifische Wechselwirkung verschiedener Inhaltsstoffe der erfindungsgemäßen Zusammensetzung mit den Oberflächenproteinen, so dass die erfindungsgemäße Zusammensetzung eine relativ breitgefächerte Wirksamkeit entfaltet. Weiterhin beeinflusst die erfindungsgemäße Zusammensetzung vermutlich auch Vermehrungsprozesse der Viren innerhalb der Zelle.

Darüber hinaus induziert die erfindungsgemäße Zusammensetzung vermutlich eine Vielzahl immunologischer Abwehrmechanismen. Hierzu zählt beispielsweise die Aktivierung von antigenspezifischen T-Lymphozyten durch Inhaltsstoffe der erfindungsgemäßen Zusammensetzung. Diese aktivierten T-Lymphozyten greifen direkt die virusinfizierten Zellen an. Weiterhin kann von diesen aktivierten T-Lymphozyten Interferon-Gamma (IFNγ), ein für die Entstehung und Aufrechterhaltung entzündlicher Prozesse wichtiges Zytokin, freigesetzt werden. Dieser Botenstoff verstärkt zum einen wiederum die Aktivierung der T-Lymphozyten und aktiviert darüber hinaus auch die natürlichen Killer-Zellen. Auch diese Zellen greifen wiederum die virusinfizierten Zellen an. Zusätzlich kann das Interferon-Gamma direkt auf die Virusvermehrung innerhalb der infizierten Zellen hemmend wirken. Insgesamt wirkt die erfindungsgemäße Zusammensetzung aktivierend auf das Immunsystem und setzt eine Vielzahl von Mechanismen in Gang, die die Virusvermehrung und die virusinfizierten Zellen bekämpfen.

Neben der akuten Bekämpfung der Viren wirkt die erfindungsgemäße Zusammensetzung ebenfalls gegen eine erneute Aktivierung der latenten Viren. Diese besonders vorteilhafte Wirkung der erfindungsgemäßen Zusammensetzung beruht vermutlich auch auf den sich gegenseitig unterstützenden antiviralen und immunologischen Effekten der Zusammensetzung. Diese synergistischen Effekte sind vermutlich die Ursache für die überraschenden therapeutischen Erfolge der erfindungsgemäßen Zusammensetzung.

Durch die sehr unterschiedlichen Angriffspunkte der Zusammensetzung, die sich gegenseitig fördern, wird weiterhin vorteilhafterweise die Ausbildung von Resistenzen der jeweiligen Erreger vermieden. Solche sich entwickelnden Resistenzen stellen insbesondere im Krankenhausalltag ein sehr großes Problem dar. Dies trifft vor allem für chemisch definierte Präparate zu, die anfangs sehr wirksam sein können, aber später überhaupt keine Wirkung mehr entfalten, da die entsprechenden Erreger infolge von Mutationen oder ähnlichem nicht mehr auf dieses Präparat ansprechen. Bei der Verwendung der erfindungsgemäßen Zusammensetzung ist vorteilhafterweise die Entwicklung solcher Resistenzen aufgrund der vermutlich sehr unterschiedlichen Wirkmechanismen ausgeschlossen.

Erkrankungen, welche mit Hilfe der erfindungsgemäßen pharmazeutischen Zusammensetzung behandelbar sind, umfassen beliebige durch Mikroorganismen hervorgerufene Erkrankungen, insbesondere jedoch virale Erkrankungen. Bevorzugt können unter Verwendung der pharmazeutischen Zusammensetzung der vorliegenden Erfindung Erkrankungen der Haut und/oder einer Schleimhaut, inbesondere der Mundschleimhaut oder Nasenschleimhaut, behandelt werden. Stärker bevorzugt wird die pharmazeutische Zusammensetzung zur Behandlung von Erkrankungen eingesetzt, deren Symptome sich im Lippen- und/oder Rachenraum äußern, wobei die Behandlung von Erkrankungen, welche durch Herpes-Viren, insbesondere Herpes labialis, verursacht werden, am stärksten bevorzugt ist. Allerdings sind die mit der erfindungsgemäßen Zusammensetzung behandelbaren Erkrankungen keineswegs auf Herpes-Erkrankungen beschränkt. So können auch andere Erkrankungen, welche auf viralen oder bakteriellen Infektionen beruhen, insbesondere Erkältungskrankheiten oder grippale Infekte, sehr wirksam mit der pharmazeutischen Zusammensetzung der vorliegenden Erfindung therapiert werden.

In einem zweiten Aspekt betrifft die vorliegende Erfindung die Verwendung von Süßholzwurzel und Eichenrinde in Kombination mit Dexpanthenol zur Herstellung einer pharmazeutischen Zusammensetzung zur Behandlung von Erkrankungen, welche durch Mikroorganismen, und insbesondere Viren, hervorgerufen werden. Bezüglich dieser Verwendung wird auf vorstehende Ausführungen verwiesen.

Die vorliegende Erfindung betrifft ferner die Verwendung eines Extrakts aus Süßholzwurzel und Eichenrinde in Kombination mit Dexpanthenol als Kosmetikum. Das Kosmetikum dient dem Schutz, der Pflege, der Reinigung und/oder Verbesserung der Beschaffenheit der Haut und/oder von Schleimhaut, wobei eine Anwendung des Kosmetikums insbesondere im Anschluss an eine virale oder bakterielle Infektion vorteilhaft ist.

Die vorliegende Erfinderung wird durch nachfolgendes Beispiel näher erläutert.

### Beispiel

Für die Herstellung eines Extraktes werden 6,67 g getrocknete und in kleine Stücke geschnittene Süßholzwurzel und 3,33 g getrocknete und in kleine Stücke geschnittene Eichenrinde eingewogen und in einen verschließbaren Behälter eingebracht. Nach Übergießen der Drogen mit 75,94 g Ethanol (96%ig) und 91,69 g deionisiertem Wasser wurde das Gemisch für etwa 5 Minuten gerührt, der Behälter anschließend verschlossen, und der Ansatz für etwa 72 Stunden bei Raumtemperatur stehengelassen. Nach Ablauf dieser Zeit wurde der Ansatz filtriert und das Filtrat gewogen. 142,50 g des auf diese Weise erhaltenen Filtrats wurden mit 7,50 g Dexpanthenol (75%ig) versetzt, portioniert, in geeignete Gefässe abgefüllt und als pharmazeutische Zusammensetzung eingesetzt.

Diese Zusammensetzung wurde von freiwilligen Probanden im Mund- und/oder Rachenraum angewendet. Bei sich ankündigenden oder schon aufgetretenen Herpes-Bläschen wurde die Zusammensetzung mit einem Wattestäbchen auf die betroffenen Hautstellen mehrfach am Tag aufgetragen. Bei anderen Entzündungen im Mund- und/oder Rachenraum wurde die Lösung ebenfalls entweder lokal aufgetragen oder es wurde mit einer Verdünnung dieser Lösung gegurgelt.

In allen Fällen zeigte sich eine deutliche Linderung der Beschwerden. In Bezug auf eine Herpes-Erkrankung im Lippenbereich war die Wirksamkeit der erfindungsgemäßen Zusammensetzung den im Stand der Technik beschriebenen Zusammensetzungen enthaltend Süßholzwurzel und Eichenrinde im Hinblick auf Wirksamkeit bzw. Veträglichkeit deutlich überlegen und jenen mit Aciclovir zumindest gleichwertig. Darüber hinaus zeigte die erfindungsgemäße Zusammensetzung eine deutlich bessere Langzeitwirkung als Aciclovir. Nach Behandlung der akuten Herpes-Bläschen mit Aciclovir und Abheilung dieser Bläschen traten bei der herkömmlichen Behandlung bei einem Probanden sehr häufig innerhalb der folgenden 14 Tage erneut Rezidive auf. Durch die Behandlung mit der erfindungsgemäßen Zusammensetzung konnten einerseits die akuten Herpes-Beschwerden deutlich gebessert werden, und andererseits die erneuten Rezidive verhindert werden. Dieser langfristige Effekt, welcher vermutlich auf die synergistischen Wirkungen der verschiedenen Inhaltsstoffe der Zusammensetzung auf das Immunsystem zurückzuführen ist, zeigt die deutlichen Vorteile der erfindungsgemäßen Zusammensetzung gegenüber herkömmmlichen Zusammensetzungen bei der Behandlung von viralen Erkrankungen.

## Patentansprüche

1. Pharmazeutische Zusammensetzung, umfassend einen Extrakt aus Süßholzwurzel und Eichenrinde in Kombination mit Dexpanthenol.

2. Pharmazeutische Zusammensetzung nach Anspruch 1, **dadurch gekennzeichnet, dass** der Gewichtsanteil der Kombination von Extrakt und Dexpanthenol etwa 1 bis etwa 100%, bezogen auf das Gesamtgewicht der pharmazeutischen Zusammensetzung, beträgt.

3. Pharmazeutische Zusammensetzung nach Anspruch 1 oder 2, **dadurch gekennzeichnet, dass** das Gewichtsverhältnis von Extrakt zu Dexpanthenol etwa 99:1 bis etwa 90:10 beträgt.

4. Pharmazeutische Zusammensetzung nach einem der Ansprüche 1-3, **dadurch gekennzeichnet, dass** als Auszugsmittel für den Extrakt eine Mischung aus Wasser und einem organischen Lösungsmittel verwendet wird.

5. Pharmazeutische Zusammensetzung nach Anspruch 4, **dadurch gekennzeichnet, dass** als organisches Lösungsmittel Ethanol verwendet wird.

6. Pharmazeutische Zusammensetzung nach Anspruch 5, **dadurch gekennzeichnet, dass** der Volumenanteil von Ethanol im Auszugsmittel etwa 20 bis etwa 80% beträgt.

7. Pharmazeutische Zusammensetzung nach einem der Ansprüche 1-6, **dadurch gekennzeichnet, dass** die Extraktion bei einer Temperatur von etwa 15 bis etwa 35°C durchgeführt wird.

8. Pharmazeutische Zusammensetzung nach einem der Ansprüche 1-7, **dadurch gekennzeichnet, dass** das Gewichtsverhältnis von Süßholzwurzel zu Eichenrinde bei der Herstellung des Extrakts etwa 5:1 bis etwa 1:3 beträgt.

9. Pharmazeutische Zusammensetzung nach einem der Ansprüche 1-8, **dadurch gekennzeichnet, dass** sie weiterhin pharmazeutisch annehmbare Träger, Feuchthaltemittel und/oder Additive umfasst.

10. Pharmazeutische Zusammensetzung nach Anspruch 9, **dadurch gekennzeichnet, dass** der pharmazeutisch annehmbare Träger als Nahrungsmittel, Genussmittel und/oder Körperpflegemittel dient.

11. Pharmazeutische Zusammensetzung nach Anspruch 9 oder 10, **dadurch gekennzeichnet, dass** das Feuchthaltemittel Propylenglykol, Pentylenglykol, Glycerin und/oder Polyethylenglykol umfasst.

12. Pharmazeutische Zusammensetzung nach einem der Ansprüche 1-11, **dadurch gekennzeichnet, dass** sie als Spray, Gel, Creme, Salbe, Lotion, Tablette oder Zäpfchen formuliert ist.

13. Pharmazeutische Zusammensetzung nach einem der Ansprüche 1-12, **dadurch gekennzeichnet, dass** sie für topische Verabreichung formuliert ist.

14. Pharmazeutische Zusammensetzung nach einem der Ansprüche 1-13, **dadurch gekennzeichnet, dass** sie zur Behandlung von Erkrankungen vorgesehen ist, welche durch Mikroorganismen hervorgerufen werden.

15. Pharmazeutische Zusammensetzung nach Anspruch 14, **dadurch gekennzeichnet, dass** es sich bei den Mikroorganismen um Viren handelt.

16. Pharmazeutische Zusammensetzung nach Anspruch 14 oder 15, **dadurch gekennzeichnet, dass** es sich bei der Erkrankung um eine Erkrankung der Haut und/oder einer Schleimhaut handelt.

17. Pharmazeutische Zusammensetzung nach einem der Ansprüche 14-16, **dadurch gekennzeichnet, dass** die Symptome der Erkrankung im Lippen- und/oder Rachenraum auftreten.

18. Pharmazeutische Zusammensetzung nach einem der Ansprüche 14-17, **dadurch gekennzeichnet, dass** die Erkrankung eine Herpes-Erkrankung ist.

19. Verwendung eines Extrakts aus Süßholzwurzel und Eichenrinde in Kombination mit Dexpanthenol zur Behandlung von Erkrankungen, welche durch Mikroorganismen hervorgerufen werden.

20. Verwendung eines Extrakts aus Süßholzwurzel und Eichenrinde in Kombination mit Dexpanthenol zur Herstellung einer pharmazeutischen Zusammensetzung zur Behandlung von Erkrankungen, welche durch Mikroorganismen hervorgerufen werden.

21. Verfahren zur Behandlung von Erkrankungen, welche durch Mikroorganismen hervorgerufen werden, **dadurch gekennzeichnet, dass** einem Patienten eine pharmazeutisch wirksame Menge eines Extrakts aus Süßholzwurzel und Eichenrinde in Kombination mit Dexpanthenol verabreicht wird.

22. Verwendung eines Extrakts aus Süßholzwurzel und Eichenrinde in Kombination mit Dexpanthenol als Kosmetikum.
